# EUROPEAN PATENT APPLICATION

(11) **EP 2 194 141 A1**
(43) Date of publication of application: **09.06.2010**
(21) Application number: 08835363.6
(22) Date of filing: 03.10.2008
(51) Int. Cl.: C12Q 1/34, G01N 33/15, G01N 33/50

(54) **METHOD OF PREDICTING DRUG-INDUCED PHOSPHOLIPIDOSIS**

(30) Priority: 04.10.2007 JP 2007261125
(71) Applicant: Kyushu University, National University Corporation, Higashi-ku Fukuoka-shi Fukuoka 812-8581 (JP); Astellas Pharma Inc., Tokyo 103-8411 (JP)
(72) Inventor: TANAKA, Yoshitaka, Fukuoka-shi Fukuoka 812-8581 (JP); IKEDA, Kazuhiko, Tokyo 103-8411 (JP)
(74) Representative: Gille Hrabal Struck Neidlein Prop Roos
(86) International application number: PCT/JP2008/068034
(87) International publication number: WO 2009/044846

(57) **Abstract**

The present invention provides a method of predicting drug-induced phospholipidosis, comprising a step of contacting a mammalian cell with a test compound, a step of measuring extracellular and/or intracellular lysosomal enzyme level or activity, or measuring intracellular LC3 level, and a step of selecting a test compound that has enhanced extracellular secretion of the enzyme or increased the protein level as a compound capable of inducing drug-induced phospholipidosis.

## Description

### Technical Field

The present invention relates to a method of predicting drug-induced phospholipidosis. More particularly, the present invention relates to a method of predicting drug-induced phospholipidosis caused by a drug candidate compound and a method of diagnosing drug-induced phospholipidosis due to an existing drug, which use abnormal transport of lysosomal enzyme or enhanced autophagy (accumulation of LC3) as an index.

### Background Art

Lipidosis involving accumulation of lipids such as phospholipids, neutral lipids, sphingomyelin and the like in tissues due to administration of drugs is also called phospholipidosis (PLsis), steatosis, sphingolipidosis and the like according to the kind of accumulated lipids, and often generically referred to as drug-induced lipidosis. It is known that many of the drugs inducing lipidosis are cationic amphiphilic drugs (CADs). With the advance in genome analysis in recent years, the value of orphan receptors as drug innovation targets has been recognized, and receptor agonists or antagonists have been developed. However, such compounds often have a CAD structure. The number of cases where development as pharmaceutical products has been discontinued is increasing due to the lipidosis inducing property. Additionally, some of the approved pharmaceuticals have been reported to cause lipidosis as side effect. Thus, the development of an efficient evaluation or prediction system of the lipidosis inducing property of drugs is urgently needed.

Phospholipidosis inducing property has been examined as an item of safety studies in the preclinical stages of drug candidate compounds. For example, a method including detecting the emergence of a myelin-like structure in the cell collected from an experimental animal administered with a test substance by an electron microscope observation (non-patent document 1), a method including utilizing a particular gene (non-patent document 2) or a metabolite (non-patent document 3) as a biomarker and the like are available. However, they are inferior in the convenience as a screening method. In addition, methods including evaluation using cultured cells and a fluorescent phospholipid probe have been reported (patent document 1, non-patent documents 4 - 7). However, defects such as inability to evaluate a compound emitting intrinsic fluorescence and the like are assumed.
As mentioned above, any conventional screening method for drug-induced phospholipidosis is insufficient in the aspects of, for example, reliability and/or rapidness and the like, and a practical screening system has not been established.
patent document 1: JP-A-2006-112947
non-patent document 1: Prog. NeuroBiol., 60: 501-12 (2000)
non-patent document 2: Toxicol. Sci., 83: 282-92 (2005)
non-patent document 3: Biochim. Biophys. Acta, 1631: 136-46 (2003)
non-patent document 4: Biochem. Pharmacol., 53: 1521-32 (1997)
non-patent document 5: Cell Biol. Toxicol., 19: 161-76 (2003)
non-patent document 6: Biochem. Pharmacol., 62: 1661-73 (2001)
non-patent document 7: Toxicol. Sci., 90: 133-41 (2006)

### Disclosure of the Invention

### Problems to be Solved by the Invention

One of the objects of the present invention is to provide a method of predicting drug-induced phospholipidosis, which is useful for toxicity screening of drug candidate compounds. Another object of the present invention is to provide a method of diagnosing drug-induced phospholipidosis as side effects due to existing pharmaceutical products.

### Means of Solving the Problems

To achieve the above-mentioned objects, the present inventors first tried to elucidate the mechanism of drug-induced phospholipidosis. In PLsis, phospholipid is mainly accumulated in lysosome, and a circular or ellipse myelin-like structure (lamellar body) is observed by an electron microscope. As a mechanism of toxicity, 1) inhibition of lysosomal enzyme (mainly phospholipid degrading enzyme (phospholipase)) activity by a drug, 2) inhibition of transport pathway relating to phospholipid metabolism by a drug, 3) inhibition of degradation of complex by formation of a drug-phospholipid complex, 4) enhanced phospholipid biosynthesis by a drug and the like have been proposed. However, the mechanism has not been elucidated.

Thus, the present inventors have examined localization of various organelle-specific proteins in the presence of a phospholipidosis inducing drug (PLsis Inducing Drugs; PLIDs) using these organelle-specific antibodies. As a result, it has been clarified that late endosome or lysosome is enlarged, and the localization of mannose 6-phosphate receptor (MPR) is altered from TGN to other organelle (endosome and the like) in the presence of PLID. MPR is generally localized in the transgolgi network (TGN), and responsible for the transport of a lysosomal enzyme biosynthesized in the rough endoplasmic reticulum to lysosome.

From the above results, it is suggested that PLsis is caused by depletion of MPR from its action site TGN and then this depletion prevents normal transport of lysosomal enzyme to lysosome, which in turn inhibits metabolism of phospholipid in the lysosome and allows accumulation thereof. In an attempt to determine the location of lysosomal enzyme not transported normally to lysosome, the present inventors examined distribution of the lysosomal enzyme in the presence of various concentrations of PLID and found that exposure to PLIDs increases extracellular lysosomal enzyme activity in a dose-dependent manner, whereas it decreases the intracellular enzyme activity. The results suggest that a decrease in the intracellular degradation capacity, which is associated with an increase in the extracellular secretion level of lysosomal enzyme, induces a delay in autophagy maturation and, as a result, causes accumulation of autophagosome and LC3 (microtuble-associated protein 1 light chain 3). Thus, the present inventors examined changes in the intracellular LC3 level in the presence of PLID by using an antibody to LC3, which is a marker protein of autophagy. As a result, it was clarified the LC3 level increases (accumulates) in the presence of PLID as compared to that in the absence thereof. Such changes in the LC3 level showed high correlation with changes in the lysosomal enzyme level in and out of the cell in the presence of PLID.

Based on such findings, the present inventors measured changes in the extracellular and/or intracellular lysosomal enzyme levels or activities, or LC3 levels due to the presence of a test substance, and successfully established a method of conveniently and rapidly predicting PLsis inducing potential of the test substance, which resulted in the completion of the present invention.

Accordingly, the present invention provides
[1] a method of predicting drug-induced phospholipidosis, comprising a step of contacting a mammalian cell with a test compound, a step of measuring extracellular and/or intracellular lysosomal enzyme level or activity, and a step of selecting a test compound that has enhanced extracellular secretion of the enzyme as a compound capable of inducing drug-induced phospholipidosis;
[2] the method of the above-mentioned [1], wherein the extracellular lysosomal enzyme level or activity is measured;
[3] a method of predicting drug-induced phospholipidosis, comprising a step of contacting a mammalian cell with a test compound, a step of measuring intracellular LC3 level, and a step of selecting a test compound that has increased an intracellular level of the protein;
[4] the method of any of the above-mentioned [1] - [3],
   wherein the mammal is selected from human, rat, mouse, hamster, monkey and dog;
[5] the method of any of the above-mentioned [1] - [4],
   wherein the cell is derived from the liver, kidney or lung, or is a lymphocyte;
[6] the method of any of the above-mentioned [1] - [5],
   wherein the cell is a cultured cell;
[7] the method of any of the above-mentioned [1], [2] and [4] - [6], wherein the lysosomal enzyme is one or more enzymes selected from the group consisting of β-hexosaminidase, β-galactosidase, β-glucuronidase, β-mannosidase, cathepsin D and cathepsin L; and
[8] a method of screening for toxicity of a drug candidate compound, comprising excluding a compound capable of inducing drug-induced phospholipidosis, which is selected by the method of the above-mentioned [1] or [3], from candidates.

Furthermore, the present invention provides [9] a test method for diagnosing drug-induced phospholipidosis or a disease associated therewith in a mammal, comprising a step of measuring a lysosomal enzyme level or activity of a sample collected from a test animal, and a step of detecting whether extracellular secretion of the enzyme has increased as compared to a control animal;
[10] a test method for diagnosing drug-induced phospholipidosis or a disease associated therewith in a mammal, comprising a step of measuring an LC3 level of a sample collected from a test animal, and a step of detecting whether an intracellular level of the protein has increased as compared to a control animal;
[11] the method of the above-mentioned [9] or [10], wherein the sample is serum, plasma or urine;
[12] a method of predicting drug-induced phospholipidosis, comprising a step of contacting a mammalian cell with a test compound, a step of examining localization of a mannose 6-phosphate receptor in the cell, and a step of selecting a test compound that has altered the localization of the receptor as a compound capable of inducing drug-induced phospholipidosis; and
[13] a test method for diagnosing drug-induced phospholipidosis or a disease associated therewith in a mammal, comprising a step of examining localization of a mannose 6-phosphate receptor in a cell collected from a test animal, and a step of assaying to determine whether the localization of the receptor has altered; and the like.

### Effect of the Invention

The method of predicting drug-induced phospholipidosis of the present invention characterized by detection of enhanced extracellular secretion of lysosomal enzyme on exposure of a mammalian cell to a compound (or measurement of intracellular LC3 level) affords an advantageous effect in that it can examine many compounds rapidly and conveniently as compared to conventional in vivo toxicity tests and evaluation methods using marker gene expression or intracellular accumulation of phospholipid and the like as an index.

### Brief Description of the Drawings

Fig. 1 shows stained images (LGP85) (Fig. 1A) and stained images (Nile Red) (Fig. 1B) after exposure of NRK cells to amiodarone (AD) for 24 hr, and stained images of LGP85 (Fig. 1C) after exposure of NRK cells cultured in a medium containing lipoprotein-deficient serum to various concentrations of AD for 24 hr.
Fig. 2 shows stained images of MPR after exposure of NRK cells to AD, chloroquine (CQ) and tilorone (TLR) for 24 hr.
Fig. 3 shows intracellular and extracellular activities of lysosomal enzymes (A: β-hexosaminidase, B: β-galactosidase, C: β-glucuronidase, D: β-mannosidase, E: cathepsin D) and endoplasmic reticulum enzyme (F: α-glucosidase) after exposure of NRK cells to various concentrations of AD for 24 hr, wherein the upper panel shows relative activity when the activity in the absence of AD is 100%, and the lower panel shows the ratio of the activity of each fraction to the total activity.
Fig. 4 shows intracellular and extracellular cathepsin D (CTD) levels (Fig. 4A) and CTD activities (Fig. 4B) after exposure of NRK cells to various concentrations of AD for 24 hr.
Fig. 5 shows intracellular and extracellular CTD levels after exposure of NRK cells to AD (lanes 2, 7), CQ (lanes 3, 8), TLR (lanes 4, 9) and ammonium chloride (lanes 5, 10) for 24 hr (lanes 1 - 5: CTD immunoprecipitate in cell lysate; lanes 6 - 10: CTD immunoprecipitate in medium).
Fig. 6 shows intracellular and extracellular cathepsin L (CTL) levels (Fig. 6A) and cathepsin B/L activities (Fig. 6B) after exposure of NRK cells to various concentrations of AD for 24 hr.
Fig. 7 shows intracellular localization of MPR and LGP85 (Fig. 7A) and β-hexosaminidase in a medium (Fig. 7B) after exposure of NRK cells to AD for various periods of time.
Fig. 8 shows intracellular LC3 levels (Fig. 8A) and stained images (Fig. 8B) after exposure of NRK cells to AD for various periods of time.
Fig. 9 shows LC3 levels (Fig. 9A) and quantify valued of LC3-II (Fig. 9B) after exposure of NRK cells to various PLIDs for 24 hr.

### Best Mode for Carrying out the Invention

The present invention relates to a method comprising contacting a mammalian cell with a test compound, measuring extracellular and/or intracellular lysosomal enzyme level or activity, and predicting whether the test compound can induce drug-induced phospholipidosis by using, as an index, whether the test compound enhances extracellular secretion of the enzyme.
The present invention also relates to a method comprising contacting a mammalian cell with a test compound, measuring intracellular LC3 level, and predicting whether the test compound can induce drug-induced phospholipidosis by using, as an index, whether the test compound increases the intracellular protein level.

Examples of the compound to be subjected to a test using the method of the present invention include candidate compounds of a drug or an animal drug, and the like. Particularly, since many samples can be treated rapidly, application to many candidate compound groups synthesized in the initial stages of drug discovery is preferable. In this case, as "a mammalian cell", a sample containing a cell or non-human mammal individual is used. On the other hand, since the level and activity of lysosomal enzyme, and LC3 level can be measured using a sample permitting easy sampling such as blood and the like, the method can be preferably used in the final stages of drug development such as preclinical test and clinical test.

Examples of available mammalian cell-containing sample include any cells of mammal (e.g., human, monkey, bovine, horse, pig, sheep, goat, dog, cat, rabbit, hamster, guinea pig, mouse, rat etc.), desirably a mammal to be the subject of administration of a test compound [for example, hepatocytes, splenocytes, nerve cells, glial cells, P cells of pancreas, bone marrow cells, mesangial cells, Langerhans' cells, epidermic cells, epithelial cells, goblet cells, endothelial cells, smooth muscle cells, fibroblasts, fibrocytes, myocytes, adipocytes, immunocytes (e.g., macrophages, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, interstitial cells, or corresponding precursor cells, stem cells, cancer cells, and the like], or from any tissues where these cells are present [for example, brain, brain regions (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, reproductive gland, thyroid, gall-bladder, bone marrow, adrenal gland, skin, lung, gastrointestinal tract (e.g., large intestine, small intestine), blood vessels, heart, thymus, spleen, submandibular gland, peripheral blood, prostate, testis, ovary, placenta, uterus, bone, joints, adipose tissue, skeletal muscles and the like], cultured cells (cell lines) established from the above-mentioned cells or tissues, and the like. Preferred are hepatocytes, kidney cells, monocytes, peripheral blood lymphocytes, fibroblasts, adrenal gland steroid producing cells, testis cells, ovary cells, abdominal cavity macrophages, alveolar epithelial cells, bronchusepithelial cells, alveolar macrophages and the like. Because of the good reproducibility (particularly in the case of human cells), the ease of availability and the like, a culture cell is preferably used. Examples of the human cultured cell include, but are not limited to, HepG2 cell line derived from liver cancer, U-937 cell line derived from lymphoma, THP-1 cell line derived from monocytes, Caco-2 cell line derived from colorectal cancer, and the like.
On the other hand, examples of the non-human mammal include, but are not limited to, rat, hamster, guinea pig, rabbit, mouse, monkey, dog, pig, cat, sheep, goat, horse, bovine and the like. Preferred are rat, hamster, guinea pig, rabbit, mouse, monkey, dog and the like. For example, as the rat cultured cell, NRK cell line derived from kidney and the like are nonlimitatively preferable and, as the hamster cultured cell, CHL cell line derived from lung and the like are nonlimitatively preferable. NRK cell is particularly preferably used, since extracellular secretion of lysosomal enzyme remarkably increases by exposure to a phospholipidosis inducing drug and, as a result, changes in intracellular LC3 level can be detected easily.

While the method of contacting a mammalian cell-containing sample with a test compound is not particularly limited, specifically, for example, when a culture cell is used as the sample, cells in the cell proliferation phase cultured in an appropriate medium under suitable conditions are detached using trypsin-EDTA and the like and centrifuged, and the cells are collected, after which an appropriate medium [e.g., MEM medium containing about 5% to about 20% fetal bovine serum (FBS) (Science, 122: 501 (1952)), DMEM medium (Virology, 8: 396 (1959)), RPMI 1640 medium (The Journal of the American Medical Association, 199: 519 (1967)), 199 medium *(*Proceeding of the Society for the Biological Medicine, 73: 1 (1950)) and the like (antibiotics such as penicillin, streptomycin and hygromycin may further be added as necessary)] is added to suspend the cells to obtain a desired cell density. While the cell density is not particularly limited as long as lysosomal enzyme and its activity, or LC3 can be detected, it is preferable to adjust the cell density so that the cells retain the state in the cell proliferation phase. Therefore, preferable initial cell density varies depending on the growth rate of the cells used and the like, and can easily be set according to the cells used by those skilled in the art, and is normally about 10⁴ cells/mL to about 10⁷ cells/mL. The cells are cultivated under ordinary conditions, for example, in a CO₂ incubator, under an atmosphere of 5% CO₂/95% air, 5% CO₂/5% O₂/90% air and the like, at about 30°C to 40°C for about 3 hours to 168 hours, preferably about 6 hours to 48 hours, more preferably about 12 hours to 24 hours. A test compound dissolved in an appropriate solvent is further diluted with the medium and added to the cells sufficiently adhered by cultivation such that the final concentration is equal to or below the highest concentration at which the cells can survive (the final concentration can be determined by separately performing histopathological observation), and the cells are cultured under ordinary conditions, for example, in a CO₂ incubator, in an atmosphere such as 5% CO₂/95% air or 5% CO₂/5% O₂/90% air, at about 30°C to 40°C for about 3 hours to 168 hours, preferably about 6 hours to 48 hours, and more preferably about 12 hours to 24 hours.

Examples of the mammal individual include humans, monkeys, rats, mice, hamsters, guinea pigs, dogs, cats, rabbits, pigs, sheep, goats, horses, cattle and the like can be mentioned. Preference is given to humans, monkeys, dogs, rats, mice, hamsters and the like. The animal's sex, age, body weight and the like are not subject to limitation; varying depending on animal species, in the case of, for example, humans, healthy male adults are usually preferably chosen in phase I studies (except for therapeutics for diseases characteristic of females or children, anticancer agents and the like) from the viewpoint of maternal protection and the like. In the case of rats, individuals at about 2 months to 24 months of age weighing about 100 g to 700 g are preferably used, but these are not to be construed as limiting the scope of the present invention.

When the mammal is a non-human animal, it is preferable to use a genetically and microbiologically controlled population of animals. For example, it is genetically preferable to use an animal of inbred strain or closed colony; in the case of rats, inbred rats such as Sprague-Dawley (SD), Wistar, and LEW can be mentioned as examples; in the case of mice, inbred mice such as BALB/c, C57BL/6, C3H/He, DBA/2, SJL, and CBA and closed colony mice such as DDY and ICR can be mentioned, but these are not to be construed as limiting the scope of the present invention. Although the animal may be a microbiologically conventional animal, it is more preferable to use an animal of SPF (specific pathogen free) or gnotobiotic grade from the viewpoint of elimination of the influence of infectious disease.

A method of exposing a mammal individual to a test compound is not particularly limited as long as a test compound is administered to the animal such that a sufficient amount of the test compound is delivered to the target cell (hepatocyte, kidney cell, monocyte, peripheral blood lymphocyte, fibroblast, adrenal gland steroid-producing cell, testis cell, ovary cell, abdominal cavity macrophage, alveolar epithelial cell, bronchial epithelial cell, alveolar macrophage etc.). For example, the test compound can be administered orally or parenterally (e.g., intravenous, intramuscular, intraperitoneal, intra-arterial, subcutaneous, intradermal, intratracheal and the like) in the form of solid, semi-solid, liquid, aerosol and the like. The dose of the test compound varies depending on the kind of compound, animal species, body weight, dosage form and the like; for example, a dose required to expose the animal to the test compound at the highest concentration allowing the target cells to survive for a given time or longer, as long as the animal can survive, and the like can be mentioned. In clinical studies, various doses are selected within the range established on the basis of the data obtained in pre-clinical studies. Administration can be performed at one time or in several divided doses. Time from administration to sample collection varies depending on the animal species, dose of the test compound, drug disposition and the like; in the case of, for example, rats, when a high dose is administered for a short time, about 1 day to 7 days, preferably about 3 days to 5 days, from initial administration can be mentioned. When a low dose is administered for a long time, about 1 month or more, preferably about 2 months to 6 months, from initial administration can be mentioned.

The animal husbandry concerning feeding, watering, bright/dark phase cycling and the like during the administration period, is not subject to limitation; in the case of rats, mice and the like, for example, a method comprising rearing the animals having free access to a commercially available solid or powder food and fresh tap water or well water in a 12-hour light/dark cycle can be mentioned. The animals may be fasted and/or water-denied for a given period as necessary.

Preferable examples of the sample collected from an experimental animal administered with a test compound include those containing various cells exemplified for the mammalian cell-containing sample and the like. Particularly preferred are blood (e.g., peripheral blood) and liquid fractions thereof (e.g., serum, plasma), and body fluids such as urine, lymph fluid, semen and the like, since they can be collected rapidly and conveniently, and are less-invasive to the animal and the like.

In the prediction method of the present invention, the extracellular and/or intracellular lysosomal enzyme levels or activities, or intracellular LC3 levels of a mammalian cell contacted with a test compound are measured. Here, the "lysosomal enzyme" means any enzyme generally localized in lysosome and involved in the metabolism of substance containing lipid, which is accumulated in lysosome. Examples thereof include, but are not limited to, β-hexosaminidase, β-galactosidase, β-glucuronidase, β-mannosidase, cathepsin D, cathepsin L and the like. The lysosomal enzyme to be a measurement target may be one or two or more. When the enzyme activity is an index, a measurement target more preferably contains highly sensitive β-hexosaminidase. The "LC3" is a protein involved in the degradation of the cytoplasm component by autophagy, and LC3 immediately undergoes C-terminal processing after translation (pro LC3) to become LC3-I. Further, LC3-I is amide bonded to phosphatidylethanolamine (PE), and phosphatidylethanolaminated LC3 (LC3-II) is localized in autophagosome. When protein level of LC3 is an index, therefore, LC3-II is preferably used as a measurement target.

When a mammalian cell is provided as a cell-containing sample (i.e., cell or tissue culture), the cells/tissue and/or the culture supernatant are/is collected by centrifugation, filtration and the like as appropriate, and the culture supernatant can be assayed as is, or after undergoing a treatment such as concentration as necessary, and the cells/tissue can be assayed for lysosomal enzyme level or activity or LC3 level after being prepared as a soluble fraction according to an ordinary method of extraction. For example, the mammalian cell or tissue culture can be obtained by disrupting the cells/tissue in a buffer solution for extraction such as ice-cooled phosphate buffer solution, Tris-HCl buffer solution, acetate buffer solution, or borate buffer solution, using sonication, a surfactant and the like as required, centrifuging the solution, and collecting the supernatant.
On the other hand, when a mammalian cell is provided as an animal individual, the cell-containing sample such as blood and the like collected from the animal as mentioned above is separated into a cell fraction (in the case of blood, blood cell and the like) and a liquid fraction (in the case of blood, serum or plasma), and an extract is collected from the cell fraction in the same manner as above, whereby a sample can be prepared. Alternatively, when the extracellular lysosomal enzyme level or activity alone is to be measured, a cell-free body fluid such as external fluid of a target cell and the like can be collected to give a sample.

As a method of measuring lysosomal enzyme or LC3 (hereinafter sometimes to be indicated as a "protein of the present invention"), western blotting and various immunoassays using an antibody against each enzyme protein (hereinafter sometimes to be indicated as an "antibody of the present invention") can be used. Specific examples include (i) a method comprising quantifying the protein of the present invention in a sample by competitively reacting the antibody of the present invention with a sample and a labeled protein of the present invention, and detecting the labeled protein bound to the antibody, (ii) a method comprising quantifying the protein of the present invention in a sample liquid by reacting the sample with the antibody of the present invention insolubilized on a carrier and another antibody of the present invention labeled, simultaneously or serially, and then determining the amount (activity) of the label on the insolubilizing carrier and the like.
In the quantitation method (ii) above, the two kinds of antibodies desirably recognize different portions of the protein of the present invention. For example, if one of the two antibodies is an antibody that recognizes an N-terminal portion of the protein of the present invention, the other antibody can be an antibody that reacts with a C-terminal portion of the protein of the present invention.

As examples of the labeling agent, a radioisotope, enzyme, fluorescent substance, luminescent substance and the like are used. As examples of the radioisotope, [¹²⁵I], [¹³¹I], [³H], [¹⁴C] and the like are used. As the above-described enzyme, stable one of high specific activity is preferable; for example, alkaline phosphatase, peroxidase, malate dehydrogenase and the like are used. As examples of the fluorescent substance, fluorescamine, fluorescein isothiocyanate and the like are used. When a test compound emits intrinsic fluorescence, a fluorescent substance having a different excitation wavelength and a different fluorescence wavelength needs to be selected. As examples of the luminescent substance, luminol, luminol derivative, luciferin, lucigenin and the like are used. Furthermore, a biotin-(strepto)avidin system can also be used to link an antibody or an antigen and a labeling agent.

The quantitation of the protein of the present invention using the antibody of the present invention is not subject to limitation, and any method of measurement can be used, as long as it is a measurement method wherein the amount of antibody, antigen or antibody-antigen complex corresponding to the amount of antigen in the sample is detected by a chemical or physical means and is applied to a standard curve generated using standard solutions containing known amounts of antigen. For example, nephelometry, the competitive method, the immunometric method and the sandwich method are preferably used; it is particularly preferable, in terms of sensitivity and for example, to use the sandwich method described below.

In insolubilizing the antigen or antibody, physical adsorption may be used, and a chemical bond in common use to insolubilize or immobilize a protein or an enzyme or the like, may also be used. As the carrier, insoluble polysaccharides such as agarose, dextran and cellulose, synthetic resins such as polystyrene, polyacrylamide and silicone, glass and the like can be mentioned.

In the sandwich method, the amount of the protein of the present invention in a sample can be quantified by reacting the sample solution to an antibody of the present invention insolubilized (primary reaction) and further reacting to another antibody of the present invention labeled (secondary reaction), and thereafter measuring the (amount) activity of the labeling agent on the insolubilizing carrier. The primary reaction and the secondary reaction may be conducted in the reverse order, and may be conducted simultaneously or after a time lag. The labeling agent and the method of insolubilization can be based on those described above. Also, in the immunoassay by the sandwich method, the antibody used as the antibody for a solid phase or the antibody for labeling needs not always be one kind; a mixture of two kinds or more of antibodies may be used for the purposes of measurement sensitivity improvement and the like.

The antibody of the present invention can be used for a measurement system other than the sandwich method, for example, the competitive method, the immunometric method or nephelometry and the like.
In the competitive method, the protein of the present invention and the labeled protein in the sample are competitively reacted with the antibody, after which the unreacted labeled antigen (F) and the antibody-bound labeled antigen (B) are separated (B/F separation), the amount labeled of either B or F is measured, and the protein of the present invention in the sample is quantified. For this reaction method, the liquid phase method, wherein a soluble antibody is used as the antibody and B/F separation is conducted using polyethylene glycol, a second antibody against the above-described antibody (first antibody), and the like, and the solid phase immobilization method, wherein a solid-phase-immobilized antibody is used as the first antibody (direct method) or the first antibody used is a soluble one and a solid-phase-immobilized antibody is used as the second antibody (indirect method), can be used.

An immunometric method comprises competitively reacting the protein of the present invention in a sample and a solid phased protein with a given amount of a labeled antibody, and separating a solid phase from a liquid phase, or reacting the protein of the present invention in a sample with an excess amount of a labeled antibody, adding a solid phased protein to allow unreacted labeled antibody to be bound to a solid phase, and separating a solid phase from a liquid phase. Then, the amount of label of either phase is measured and the amount of antigen in the sample is quantified.

Also, in nephelometry, the amount of insoluble precipitate resulting from an antigen-antibody reaction in the gel or in the solution is measured. Even when the amount of the protein of the present invention in the sample is small and only a small amount of precipitate is obtained, laser nephelometry, which utilizes laser scattering, and the like are preferably used.

In applying these individual immunological measurement methods to the quantitation method of the present invention, it is unnecessary to set special conditions, procedures and the like. Making ordinary technical considerations for those skilled in the art to the ordinary conditions and procedures in each method, a measurement system for the protein of the present invention can be constructed. For details of these general technical means, review, books and the like can be referred to.
For example, "Meth. Enzymol.", Vol. 70 (Immunochemical Techniques (Part A)), ibidem, Vol. 73 (Immunochemical Techniques (Part B)), ibidem, Vol. 74 (Immunochemical Techniques (Part C)), ibidem, Vol. 84 (Immunochemical Techniques (Part D: Selected Immunoassays)), ibidem, Vol. 92 (Immunochemical Techniques (Part E: Monoclonal Antibodies and General Immunoassay Methods)), ibidem, Vol. 121 (Immunochemical Techniques (Part I: Hybridoma Technology and Monoclonal Antibodies)) (all published by Academic Press) and the like can be referred to.
Using the antibody of the present invention as described above, the amount of the protein of the present invention in and out of the cell can be quantified with high sensitivity.

On the other hand, the activity of lysosomal enzyme can be measured using an activity measurement method by known per se for each enzyme. For example, lysosomal enzyme can be quantified by contacting a sample with a substrate compound for a lysosomal enzyme, which emits fluorescence or develops color by an enzyme reaction, and measuring the intensity of the resulting fluorescence or developed color by a fluorometer, spectrophotometer and the like. For example, as a measurement method of β-hexosaminidase, β-galactosidase, β-glucuronidase or β-mannosidase, the method described in The EMBO Journal, 12: 5219-5223 (1993) can be mentioned, as a measurement method of cathepsin D, the method described in J. Biochem, 125: 1137-1143 (1999) can be mentioned, and as a measurement method of cathepsin B/L, the method described in Methods in Enzymology, 80: 535-561 (1981) can be mentioned.
When the lysosomal enzyme is a protease such as cathepsin and the like, the enzyme is transported as an inactive proenzyme to lysosome, where it is often processed into an active mature protein. Thus, the protease extracellularly secreted for the induction of phospholipidosis of the cell is a proenzyme. Therefore, when a lysosomal enzyme is a protein that goes through an inactive precursor, the lysosomal enzyme level needs to be measured using an antibody, or the enzyme needs to be processed into a mature form by a method known per se, and then subjected to an activity measurement.

When a test compound induces drug-induced phospholipidosis in a mammalian cell, extracellular secretion of lysosomal enzyme increases as the localization of MPR, which is a lysosomal enzyme receptor, alters. As a result of the above-mentioned measurement, therefore, when the extracellular lysosomal enzyme level or activity has significantly increased in the presence of a test compound as compared to that in the absence of the test compound, and/or when the intracellular lysosomal enzyme level or activity has significantly decreased in the presence of a test compound as compared to that in the absence of the test compound, the test compound can be predicted to have high possibility of inducing drug-induced phospholipidosis. When a lysosomal enzyme showing a comparatively small increase in the extracellular secretion while inducing drug-induced phospholipidosis is a measurement target, extracellular and intracellular lysosomal enzyme levels or activities are measured, and the ratio of the both is compared between in the presence and in the absence of a test compound, whereby the measurement sensitivity can be improved.

When a test compound induces drug-induced phospholipidosis in a mammalian cell, since extracellular secretion of lysosomal enzyme increases, the intracellular degradation system is impaired, thereby delaying maturation of autophagy and increasing intracellular LC3 concentration. As a result of the above-mentioned measurement, therefore, when the intracellular LC3 level has significantly increased in the presence of a test compound as compared to that in the absence of the test compound, the test compound can be predicted to have high potential of inducing drug-induced phospholipidosis.

As mentioned above, the present invention is based on the finding that drug-induced phospholipidosis can be predicted using, as an index, increased extracellular secretion of lysosomal enzyme or intracellular accumulation of LC3. The method of the present invention can be used for the diagnosis of drug-induced phospholipidosis. Accordingly, the present invention also provides a test method for diagnosing drug-induced phospholipidosis or a disease associated therewith in a test mammal, comprising measuring a lysosomal enzyme level or activity or LC3 level in a sample collected from the test mammal, and assaying whether extracellular secretion of lysosomal enzyme or intracellular LC3 level has increased as compared to a control animal. Here, all of the mammal, the sample collected from the mammal is the same as those described with regard to the above-described prediction method of the present invention. The term "diagnosis" as used herein refers to a concept encompassing all diagnoses, including not only the judgment on the presence or absence of suffering, but also the determinations of severity (degree of progression), likelihood of suffering/development of disease in the future, and the like after an established diagnosis is made.

The term "drug" as used herein encompasses drugs approved and used as pharmaceuticals or animal drugs, as well as optionally chosen drugs erroneously taken, or environmentally absorbed, by the test animal, and the like.

In addition, pulmonary fibrosis, blindness, encephalopathy and the like can be mentioned as examples of diseases (adverse drug reaction symptoms) related to drug-induced phospholipidosis, but these are not to be construed as limiting the scope of the present invention. As examples of the disease in a non-human mammal, hepatic lipidosis and the like in companion animals such as cats and dogs can also be mentioned.

The present invention is based on the finding that, in drug-induced phospholipidosis, lysosomal enzyme is not normally transported to lysosome due to alterations in intracellular MPR localization, which in turn increases extracellular secretion. Therefore, the present invention also provides a method of predicting whether a test compound can induce drug-induced phospholipidosis, comprising contacting a mammalian cell with a test compound, examining localization of MPR in the cell, and predicting based on alterations in the localization of the receptor, as well as a test method for diagnosing drug-induced phospholipidosis or a disease associated therewith in a mammal, comprising examining localization of MPR in a cell collected from a test animal, and assaying to determine whether the localization of the receptor has altered. The intracellular localization of MPR can be examined by a method known per se, such as immunohistochemical staining using anti-MPR antibody and the like. As a result, when localization of MPR has altered from TGN, its inherent action site, to other organelle (endosome and the like), it is predicted (diagnosed) that a test compound has high possibility of inducing drug-induced phospholipidosis, or a test animal is affected with drug-induced phospholipidosis or a disease associated therewith, or has high possibility of being affected therewith.

### Examples

The present invention is explained in more detail in the following by referring to Examples, which are mere exemplification and do not limit the scope of the present invention.

### Reference Example 1 Swelling of late endosome/lysosome and accumulation of phospholipid by exposure to AD

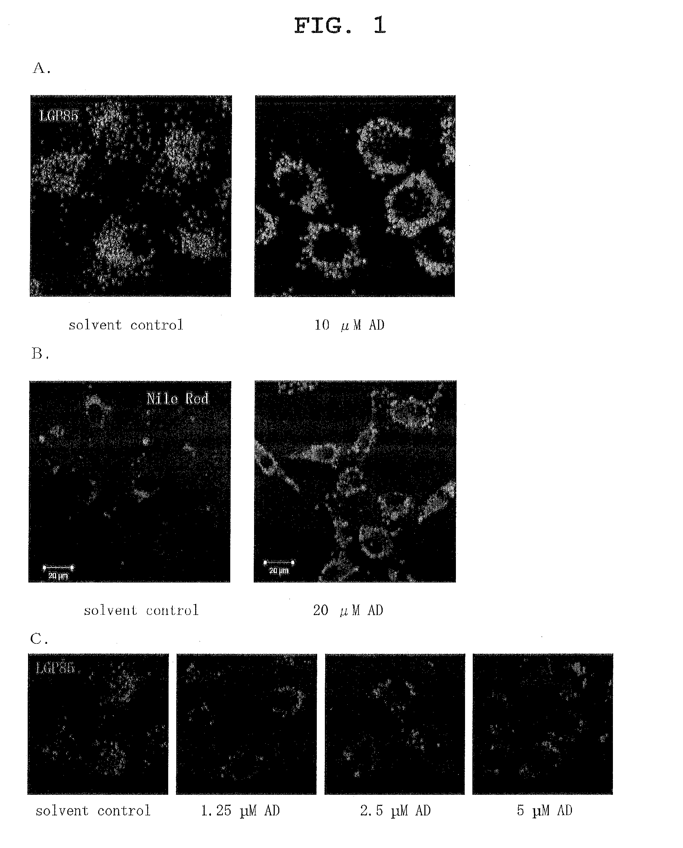

NRK cells (normal rat kidney-derived cell line) were exposed to amiodarone (AD), which is a phospholipidosis inducing drug (PLID), for 24 hr, and observed by a confocal laser microscope using an antibody to LGP85, which is a membrane protein localized in late endosome/lysosome. As a result, the organelles thereof were found to have enlarged (Fig. 1A). In addition, accumulation of phospholipid was found at the site by Nile Red staining (Fig. 1B).
The cells were cultured in a DMEM medium supplemented with lipoprotein-deficient serum to 10% instead of normal fetal bovine serum, and morphological changes of lysosome and the presence or absence of phospholipid accumulation due to exposure to AD under the conditions free of extracellular supply of lipoprotein were examined. As a result, enlargement of late endosome/lysosome occurred in an AD dose-dependent manner (Fig. 1C), and accumulation of phospholipid was found at the site. Therefore, it was suggested that phospholipid accumulated by exposure to AD was not extracellularly supplied but was an induction of phospholipidosis.

### Example 1 Changes of localization of MPR by exposure to AD

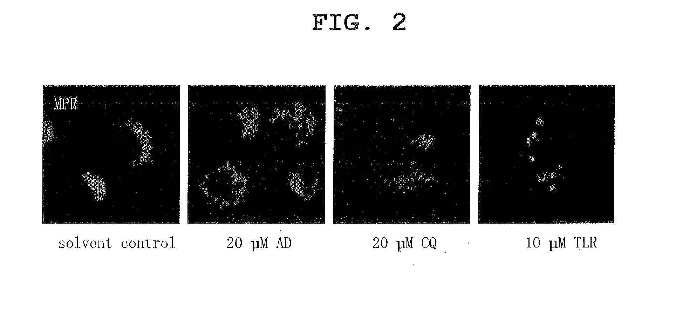

NRK cells were exposed to various concentrations of AD for 24 hr, and were observed by a confocal laser microscope using an antibody to a protein localized in late endosome/lysosome, golgi apparatus, TGN and the like in those organelles. As a result, it was clarified that the localization pattern of MPR altered by exposure to AD from the normal TGN localization, which is observed in the absence of exposure, to a large spherical vesicles (Fig. 2). Similar alterations of localization of MPR were also observed by exposure to chloroquine (CQ), tilorone (TLR) and ammonium chloride.

### Example 2 Measurement of extracellular and intracellular lysosomal enzyme activity after exposure to AD

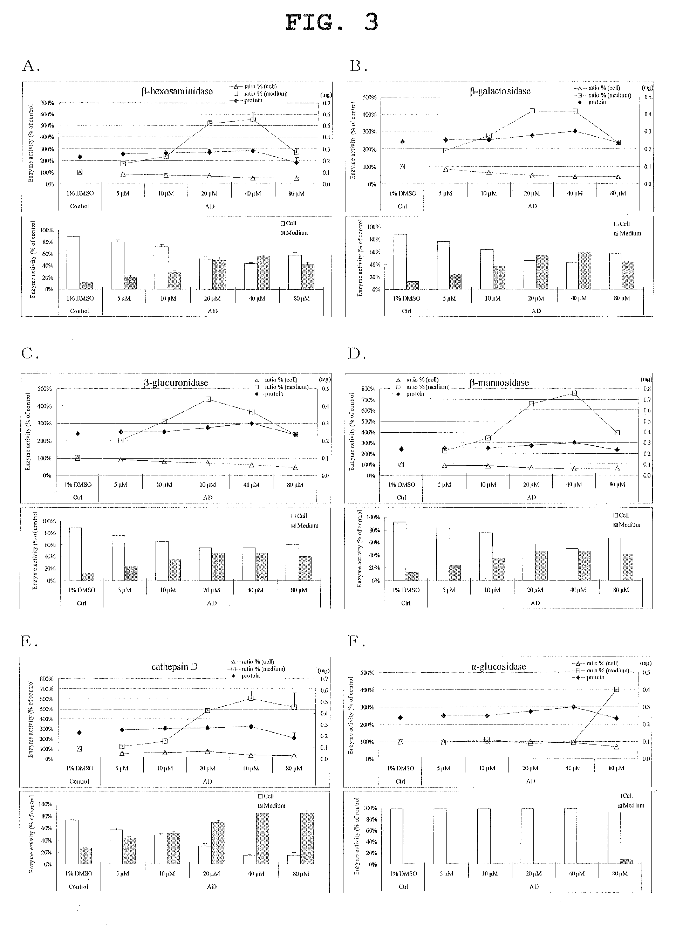

NRK cells were exposed to 0 (solvent control), 5, 10, 20, 40 and 80 µM of AD for 24 hr, and lysosomal enzyme activities in the cell and medium were measured by a conventional method. As a result, it was clarified that lysosomal enzyme activity in the medium increased in an AD dose-dependent manner and intracellular enzyme activity decreased (Fig. 3A - E). On the other hand, since the activity of α-glucosidase, which is an endoplasmic reticulum enzyme, changed only at 80 µM, at which AD shows cytotoxicity (Fig. 3F), it was established that increased lysosomal enzyme activity in the medium was not caused by extracellular leakage due to cell damage, but by selective extracellular secretion of lysosomal enzyme.

### Example 3 Increase of extracellular lysosomal enzyme activity by exposure to various PLIDs

NRK cells were exposed to 18 kinds of drugs described in Table 1 (of which 15 kinds have been reported to show in vitro or in vivo phospholipidosis inducing property) for 24 hr and the β-hexosaminidase activity in the medium was measured by a conventional method. As a result, lysosomal enzyme activity in the medium increased with 14 kinds of phospholipidosis inducing drugs other than gentamicin, and almost all compounds showed correlation with enlargement of late endosome/lysosome and alterations of localization of MPR (Table 1). When a similar experiment was performed using HeLa cells, the lysosomal enzyme activity in the medium also increased. However, the increase was approximately 1.7-fold at maximum that of the control, and it was suggested an increase in the enzyme activity can be detected with higher sensitivity by using NRK cell.

**Table 1**

| compounds | indication | range of concentration | β-hex activity increase | alteration of MPR localization |
|---|---|---|---|---|
| amiodarone^{a)} | antiarrhythmic drug | 5 - 80 µM | >10 µM | >10 µM |
| chloroquine^{a)} | antimalarial drug | 5 - 80 µM | >5 µM | >5 µM |
| haloperidol ^{a}) | antipsychotic agents | 10 - 40 µM | >10 µM | >20 µM |
| imipramine^{a)} | antidepressant | 10 - 100 µM | >40 µM | >40 µM |
| chlorpromazine^{a)} | antipsychotic agents | 5-40 µM | 20 µM | - |
| perhexiline^{a)} | anti-angina pectoris drug | 1.25 - 100 µM | 5 µM | 2.5 µM |
| maprotiline^{a)} | antidepressant | 2.5 - 100 µM | >20 µM | 20 µM |
| tamoxifen^{a)} | anti-estrogen drug | 2.5 - 100 µM | >10 µM | 10 µM |
| tilorone^{a)} | interferon inducing substance | 2.5 - 100 µM | >2.5 µM | >5 µM |
| thioridazine^{a)} | antipsychotic agents | 2.5 - 100 µM | 10 µM | 10 µM |
| quinidine^{a)} | antiarrhythmic drug | 10 - 100 µM | 100 µM | 100 µM |
| erythromycin^{a)} | macrolide antibiotic | 100 µM - 1.5 mM | >400 µM | - |
| gentamicin | aminoglycoside antibiotic | 1 - 4 mM | none | none |
| U18666A^{a)} | OSC inhibitor | 3 µg/mL | 3 µg/mL | 3 µg/mL |
| NH₄Cl^{a)} | | 1 - 50 mM | >1 mM | >5 mM |
| amoxicillin | β lactam antibiotic | 100 µM - 2 mM | none | none |
| aspirin | anti-inflammatory drug | 100 µM - 2 mM | none | none |
| captopril | ACE inhibitor | 100 µM - 2 mM | none | none |

| | | | | |
|---|---|---|---|---|
| ^{a)} drug reported to show in vitro or in vivo phospholipidosis inducing property | | | | |

### Example 4 Measurement of intracellular and extracellular lysosomal enzyme levels after exposure to AD

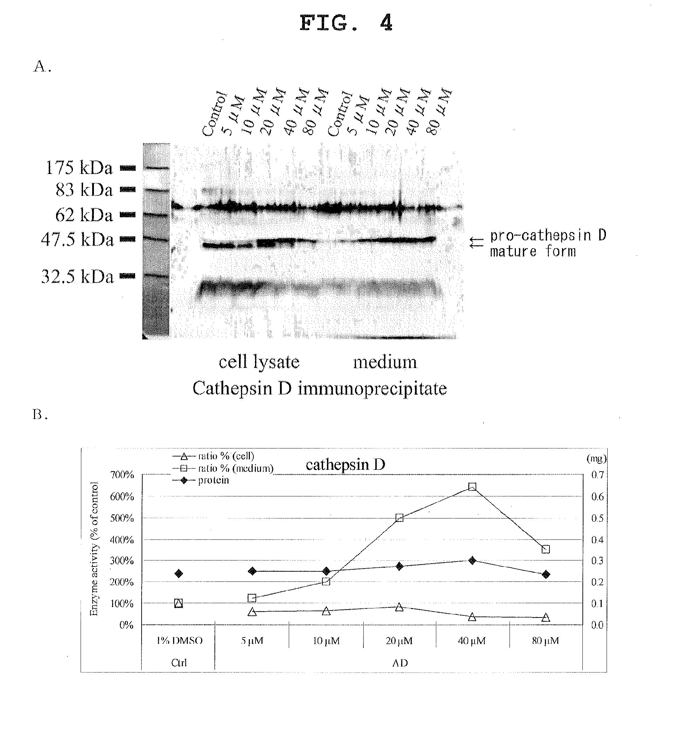

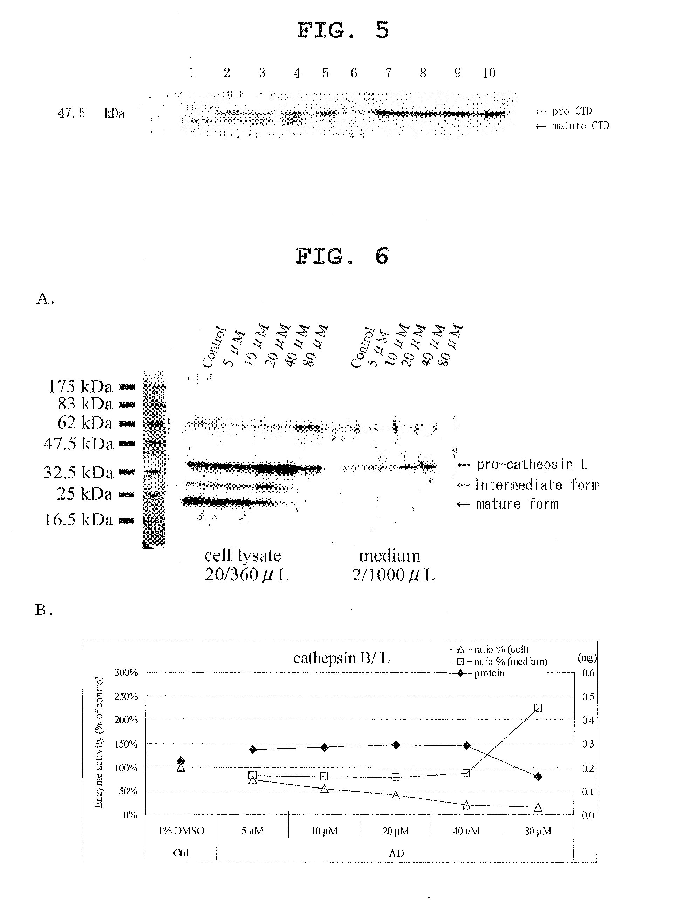

Protease such as cathepsin takes form of inactive proenzyme during transport from the golgi apparatus to lysosome, and processed in the lysosome to be active mature enzyme. In the case of cathepsin D (CTD), it is biosynthesized as an about 45 kDa proenzyme in rat cell (NRK), and processed in the acidic environment of lysosome to become an about 43 kDa mature enzyme.
Therefore, NRK cells were exposed to 0 (control), 5, 10, 20, 40 and 80 µM of AD for 24 hr, and lysosomal enzyme (CTD and cathepsin L (CTL)) levels in the cell and medium were measured by Western blotting using an antibody to these enzymes. To be specific, immunoprecipitates against anti-CTD antibody in cell lysate and medium or the cell lysate and medium for CTL were subjected to Western blot analysis using an anti-CTD antibody or anti-CTL antibody. As a result, it was shown that almost all intracellular CTD was in the form of mature enzyme in the control, whereas, by exposure to AD, the ratio of proenzyme increased in a dose-dependent manner up to the concentration of 40 µM where the cytotoxicity is low, and CTD was not normally transported to the lysosome. In addition, mature enzyme was completely absent in the medium, and only proenzyme increased in an AD dose-dependent manner (Fig. 4A). Therefore, it was also shown that increased lysosomal enzyme in the medium was not caused by extracellular leakage due to cell damage and the like, but by selective extracellular secretion thereof. Moreover, it was clarified that even when NRK cells were exposed to CQ, TLR and ammonium chloride for 24 hr, the ratio of intracellular proenzyme increased as compared to the control, and the amount of proenzyme secreted in the medium increased (Fig. 5).
On the other hand, CTL is biosynthesized as an about 39 kDa proenzyme, and converted to a 30 kDa single strand intermediate enzyme and a 23 kDa and 7 kDa double stranded mature enzyme during the process of intracellular transport. The intracellular CTL activity decreased in a dose-dependent manner due to the exposure to AD, whereas the CTL activity in the medium changed only at 80 µM where the cytotoxicity is remarkable (Fig. 6B). As a result of Western blotting using an anti-CTL antibody, however, the ratio of mature enzyme in the cell decreased in an AD dose-dependent manner, and the ratio of proenzyme increased. Moreover, mature enzyme was completely absent in the medium, and only proenzyme increased in an AD dose-dependent manner (Fig. 6A). While CTD increased the enzyme activity in the medium in an AD dose-dependent manner, CTL caused no change. The reason therefor is considered to be the measurement of CTD activity at pH 4.0 where autocatalytic processing occurs, and the measurement of CTL activity at pH 6.0 where processing scarcely occurs.

### Example 5 Time dependency of alterations of localization of MPR and extracellular secretion of lysosomal enzyme

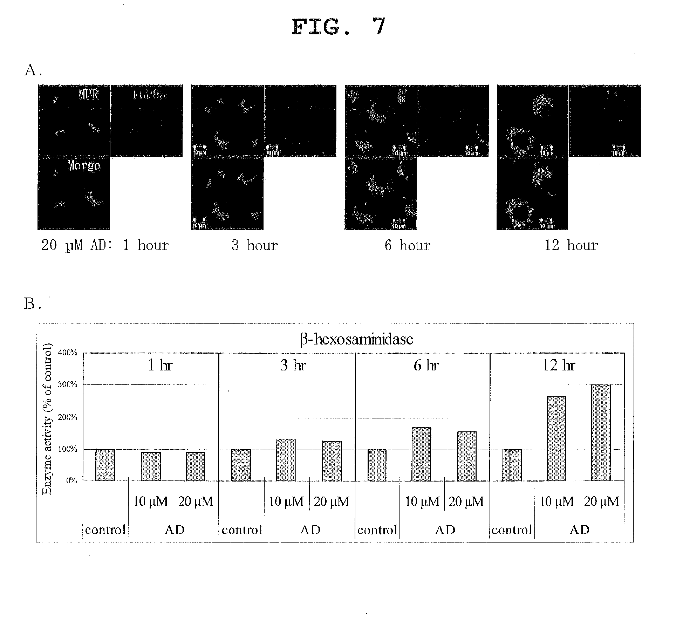

The localization of MPR and LGP85 after exposure of NRK cells to 0 (solvent control), 10 and 20 µM of AD for 1, 3, 6 and 12 hr was observed by a confocal laser microscope using an antibody to each protein. Furthermore, the activity of β-hexosaminidase in the medium was measured by a conventional method. As a result, the localization of MPR altered remarkably by exposure for 6 hr (Fig. 7A), and lysosomal enzyme activity in the medium increased to about 2-fold or more that of the control by exposure for 12 hr or longer (Fig. 7B).

### Example 6 Intracellular accumulation of LC3 by exposure to AD

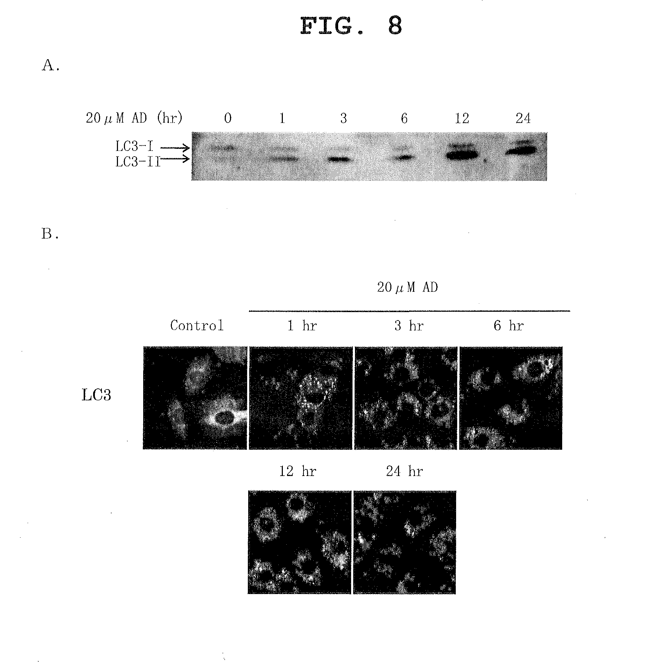

NRK cells were exposed to 20 µM of AD for 1, 3, 6, 12 and 24 hr, and the level of LC3 (microtuble-associated protein 1 light chain 3), which is an autophagy marker, in each cell was measured by Western blotting using an antibody to the protein. That is, cell lysate was subjected to Western blot analysis using an anti-LC3 antibody. As a result, the LC3-II level increased by exposure for 3 hr, and remarkably increased by exposure for 12 and 24 hr (Fig. 8A). In addition, observation by a confocal laser microscope clarified that granular staining with LC3 in the cell, which were absent without the exposure, increased in a time-dependent manner by exposure to AD (Fig. 8B).

### Example 7 Intracellular accumulation of LC3 by exposure to various PLIDs

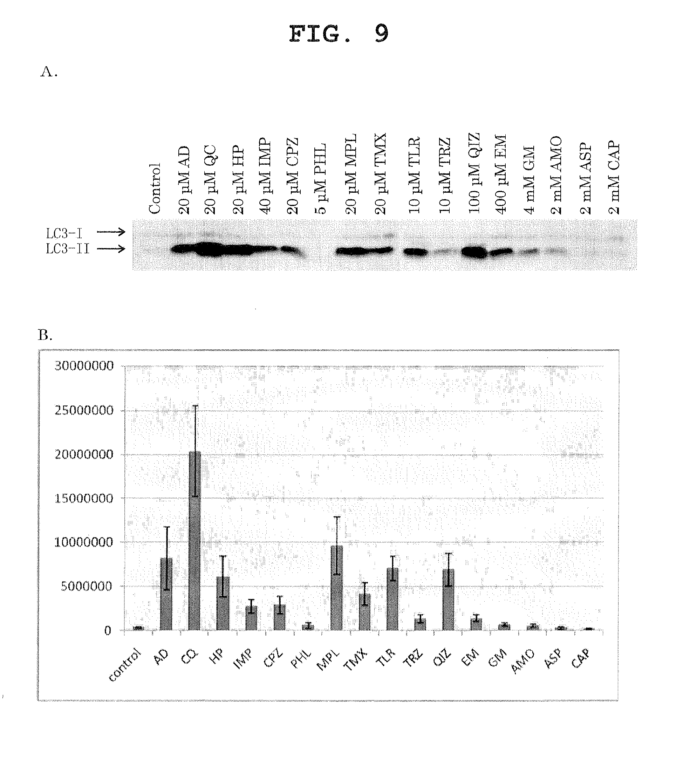

NRK cells were exposed to various PLIDs for 24 hr, and the intracellular level of LC3 was measured by western blotting. As a result, phospholipidosis inducing drug increased the intracellular LC3 level (Fig. 9A and B), and showed high correlation with an increase in the extracellular lysosomal enzyme activity.

### Industrial Applicability

The prediction method of the present invention enables convenient, rapid and highly sensitive prediction of phospholipidosis inducing property of a drug, and is useful as a screening means for rapidly distinguishing a toxic compound in early stages of development for efficient selection of a lead compound. Particularly, since the method can also evaluate even a compound with intrinsic fluorescence, for which evaluation of the presence or absence of inducing potential is assumed to be difficult by a conventional evaluation system using a fluorescent lipid probe, and the like, it is expected as an evaluation system with higher reliability.
Furthermore, since the prediction or diagnosis method of the present invention enables noninvasive diagnoses by the use of a peripheral body fluid such as plasma and the like as a sample, it is also extremely useful for clinical diagnoses of drug-induced phospholipidosis.
This application is based on a patent application No. 2007-261125 filed in Japan (filing date: October 4, 2007), the contents of which are incorporated in full herein.

## Claims

1. A method of predicting drug-induced phospholipidosis, comprising a step of contacting a mammalian cell with a test compound, a step of measuring extracellular and/or intracellular lysosomal enzyme level or activity, and a step of selecting a test compound that has enhanced extracellular secretion of the enzyme as a compound capable of inducing drug-induced phospholipidosis.

2. The method according to claim 1, wherein the extracellular lysosomal enzyme level or activity is measured.

3. A method of predicting drug-induced phospholipidosis, comprising a step of contacting a mammalian cell with a test compound, a step of measuring intracellular LC3 level, and a step of selecting a test compound that has increased an intracellular level of the protein.

4. The method according to any one of claims 1 to 3, wherein the mammal is selected from human, rat, mouse, hamster, monkey and dog.

5. The method according to any one of claims 1 to 3, wherein the cell is derived from the liver, kidney or lung, or is a lymphocyte.

6. The method according to any one of claims 1 to 3, wherein the cell is a cultured cell.

7. The method according to claim 1 or 2, wherein the lysosomal enzyme is one or more enzymes selected from the group consisting of β-hexosaminidase, β-galactosidase, β-glucuronidase, β-mannosidase, cathepsin D and cathepsin L.

8. A method of screening for toxicity of a drug candidate compound, comprising excluding a compound capable of inducing drug-induced phospholipidosis, which is selected by the method according to claim 1 or 3, from candidates.
